(19)

**Europäisches Patentamt**
**European Patent Office**
**Office européen des brevets**

(11) **EP 3 495 494 B1**

(12) **EUROPEAN PATENT SPECIFICATION**

(45) Date of publication and mention
of the grant of the patent:
**30.11.2022 Bulletin 2022/48**

(51) International Patent Classification (IPC):
*C12Q 1/68* (2018.01)    *C12N 15/09* (2006.01)
*C12Q 1/6869* (2018.01)    *C12Q 1/6886* (2018.01)

(21) Application number: **17837072.2**

(52) Cooperative Patent Classification (CPC):
**C12Q 1/68; C12Q 1/6869; C12Q 1/6886;**
C12N 15/09; C12Q 2535/101

(22) Date of filing: **03.08.2017**

(86) International application number:
**PCT/JP2017/028315**

(87) International publication number:
**WO 2018/025971 (08.02.2018 Gazette 2018/06)**

(54) **METHOD FOR DETERMINING PRESENCE OR ABSENCE OF RISK OF DEVELOPING CANCER**

VERFAHREN ZU BESTIMMUNG DER PRÄSENZ ODER ABSENZ DES RISIKOS EINER
TUMORERKRANKUNG

MÉTHODE POUR DÉTERMINER LA PRÉSENCE OU L'ABSENCE DU RISQUE DE DÉVELOPPER
UN CANCER

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **04.08.2016   JP 2016154067**

(43) Date of publication of application:
**12.06.2019   Bulletin 2019/24**

(73) Proprietors:
• **Shizuoka Prefecture**
  **Shizuoka 420-8601 (JP)**
• **Shizuoka Prefectural University Corporation**
  **Shizuoka-shi, Shizuoka 422-8526 (JP)**

(72) Inventors:
• **YAMAGUCHI, Ken**
  **Sunto-gun**
  **Shizuoka 411-8777 (JP)**
• **KUSUHARA, Masatoshi**
  **Sunto-gun**
  **Shizuoka 411-8777 (JP)**
• **SERIZAWA, Masakuni**
  **Sunto-gun**
  **Shizuoka 411-8777 (JP)**
• **MOCHIZUKI, Tohru**
  **Sunto-gun**
  **Shizuoka 411-8777 (JP)**

• **OHSHIMA, Keiichi**
  **Sunto-gun**
  **Shizuoka 411-8777 (JP)**
• **HATAKEYAMA, Keiichi**
  **Sunto-gun**
  **Shizuoka 411-8777 (JP)**
• **URAKAMI, Kenichi**
  **Sunto-gun**
  **Shizuoka 411-8777 (JP)**
• **OHNAMI, Shumpei**
  **Sunto-gun**
  **Shizuoka 411-8777 (JP)**
• **AKIYAMA, Yasuto**
  **Sunto-gun**
  **Shizuoka 411-8777 (JP)**
• **MARUYAMA, Kouji**
  **Sunto-gun**
  **Shizuoka 411-8777 (JP)**
• **INOUE, Kengo**
  **Sunto-gun**
  **Shizuoka 411-8777 (JP)**
• **SHIMODA, Yuji**
  **Sunto-gun**
  **Shizuoka 411-8777 (JP)**
• **NAGASHIMA, Takeshi**
  **Sunto-gun**
  **Shizuoka 411-8777 (JP)**

**(Cont. next page)**

EP 3 495 494 B1

- **ISHIKAWA, Yoshinobu**
  Shizuoka-shi
  Shizuoka 422-8526 (JP)

(74) Representative: **Appleyard Lees IP LLP**
15 Clare Road
Halifax HX1 2HY (GB)

(56) References cited:
WO-A1-2015/149721     WO-A1-2016/086197
WO-A2-2012/119113

- **YAMAGUCHI K: "Prevalence of low-penetrant germline TP53 D49H mutation in Japanese cancer patients", BIOMEDICAL RESEARCH (JAPAN) 2016 BIOMEDICAL RESEARCH FOUNDATION JPN, vol. 37, no. 4, 1 August 2016 (2016-08-01), pages 259-264, XP002797636, ISSN: 0388-6107**
- **LIU, Y. et al.: "Analysis of P53 mutaions and their expression in 56 colorectal cancer cell lines", PNAS, vol. 103, no. 4, 2006, pages 976-981, XP055238779, ISSN: 1091-6490**
- **KOSUKE YOSHIHARA: "The relationship between TP53 mutations and prognosis in advanced-stage serous ovarian cancers", Niigata Medical Journal, vol. 123, no. 8, 1 January 2009 (2009-01-01), pages 394-403, XP055590057, ISSN: 0029-0440**
- **NARENDRAN, A. et al.: "Mutant p53 in bone marrow stromal cells increases VEGF expression and supports leukemia cell growth", Experimental Hematology, vol. 31, 2003, pages 693-701, XP055572710, ISSN: 0301-472X**
- **HIROTSUGU KENMOTSU et al.: "Jisedai Sequencer o Mochiita Hi Shosaibo Haigan no Idenshi Kaiseki", Annual Meeting of Japan Society of Clinical Oncology Shoroku, 1 January 2014 (2014-01-01), pages 1-4, XP055590063, Retrieved from the Internet: URL:http://archive.jsco.or.jp/detail.php?s ess_id=3244 [retrieved on 2017-10-02]**
- **RIE MAKUUCHI et al.: "Bunshi Profile ni Motozuku Igan no Bunrui", Annual Meeting of Japan Society of Clinical Oncology Shoroku, 1 January 2015 (2015-01-01), pages 1-4, XP055590068, Retrieved from the Internet: URL:http://archive.jsco.or.jp/detail.php?s ess_id=5743 [retrieved on 2017-10-02]**

- **MITSUHIRO ISAKA et al.: "Project HOPE (Multiomics Kaiseki o Mochiita Gan Kanja Hyoka)-Genpatsusei Haigan Setsujo-rei no Zen Exon Kaiseki Oyobi Zen Idenshi Hatsugen Kaiseki", Annual Congress of Japan Surgical Society, April 2016 (2016-04), XP055590073, Retrieved from the Internet: URL:http://www.myschedule.jp/jss116/search /detail_program/id:232 [retrieved on 2017-10-02]**
- **ISAMU NISHISHO: "Idenshi no Shindan to Chiryo", BME, vol. 12, no. 2, 1 January 1998 (1998-01-01), pages 3-14, XP055590088,**
- **YAMAGUCHI, K. et al.: "Prevalence of low-penetrant germline TP53 D49H mutation in Japanese cancer patients", Biomedical Research, vol. 37, no. 4, 20 August 2016 (2016-08-20), pages 259-264, XP055572723, ISSN: 0976-1683**
- **J.M. Varley: "Germline TP53 mutations and Li-Fraumeni syndrome", HUMAN MUTATION, vol. 21, no. 3, 27 February 2003 (2003-02-27), pages 313-320, XP055769750, US ISSN: 1059-7794, DOI: 10.1002/humu.10185**
- **Aru Narendran ET AL: "Mutant p53 in bone marrow stromal cells increases VEGF expression and supports leukemia cell growth", EXPERIMENTAL HEMATOLOGY, vol. 31, no. 8, 1 August 2003 (2003-08-01), pages 693-701, XP055572710, US ISSN: 0301-472X, DOI: 10.1016/S0301-472X(03)00159-0**

Remarks:
The complete document including Reference Table(s) and the Sequence Listing(s) can be downloaded from the EPO website

## Description

### Technical Field

The present invention relates to a method for determining the presence or absence of a risk of developing a familial tumor. More specifically, the present invention relates to a method for determining the presence or absence of a risk of developing a familial tumor which is caused by a germ cell mutation in p53, comprising detecting the presence or absence of a mutation to substitute alanine with aspartic acid at codon 159 of human p53 (hereinafter, also referred to as "A159D mutation") and/or a mutation to substitute aspartic acid with histidine at codon 49 of human p53 (hereinafter, also referred to as "D49H mutation") in a biological sample collected from a test subject.

### Background Art

As the whole human genome has been sequenced in recent years, the presence of 3000000 or more single nucleotide polymorphisms (SNPs) differing among individuals has been revealed. There are growing needs for predicting the traits of individuals or the development of illness on the basis of information on the SNPs. Thus, the relation of SNPs to diseases is under analysis.

Owing to developed next generation DNA sequencers (NGSs) which enable reading of a wide range of genome information through one reaction using a plurality of PCR primer sets, a large number of single nucleotide mutations (SNVs) have also been identified which appear with a frequency lower than that of SNPs found with a frequency of 1% or more of the total population. Efforts aimed at achieving individualized medicine or preventive medicine based on such outcomes are also accelerating. Whole genome sequencers which identify SNV in a noncoding region including a promoter region can be deemed to be ideal for meeting the needs. At present, however, exon analysis which conducts analysis focusing on exon regions has been widely conducted in terms of cost.

On the other hand, human p53 is a protein originally having the meaning of a molecular weight of 53000. This protein consists of 393 amino acids in the whole length and is constituted by 5 regions: transactivation domains (TADs) composed of TAD1 and TAD2, a proline rich domain, a DNA binding domain (DBD), a tetramerization domain, and a regulatory domain, from the N terminus. DBD is a region involved in DNA binding, and most of gene mutations detected in tumor are focused on this region. Few gene mutations are found in TAD. The human p53 is reportedly responsible for functions of protecting the organism from gene abnormalities by a wide variety of activities. Typical examples of the activities can include the control of cell cycle progression, the activation of gene repairing enzyme, and the ability to induce apoptosis via the control of gene transcription in cells having gene abnormalities. A mechanism is considered under which a mutation in human p53 gene itself deletes these functions of the human p53, leading to the appearance of tumor. Human p53 gene mutations in human cancer cells have been confirmed in many human tumors such as large intestine, stomach, mammary gland, lung, brain, and esophageal tumors. The abnormal accumulation of varied human p53 has been observed in many tumor tissues.

Although documents regarding publicly known exhaustive variant human p53 libraries describe A159D mutation and D49H mutation (see for example, patent document 1), the relation of these mutations to illness or a particular trait is unknown.

The A159D mutation has been reported by COSMIC (URL: http://cancer.sanger.ac.uk/cosmic) as to 8 tumor tissue samples and 1 cell line and recorded under No. COSM11496. This mutation has also been reported by IRAC database as to 14 cases, and is a somatic cell mutation in all the cases. The A159D mutation is not recorded in the Human Genetic Mutation Database of Kyoto University targeting germ cell mutations, Integrative Japanese Genome Mutation Database of the Tohoku University Tohoku Medical Megabank Organization, the Exome Aggregation Consortium (URL: http://exac.broadinstitute.org) database, the dbSNP database (URL: http://www.ncbi.nlm.nih.gov/SNP/) or Clinvar (URL: https://www.ncbi.nlm.nih.gov/clinvar/). There is no report on the A159D mutation as to Li-Fraumeni syndrome, Li-Fraumeni like syndrome, or familial tumor which is caused by a germ cell mutation in p53.

The D49H mutation has been further reported by COSMIC (URL: http://cancer.sanger.ac.uk/cosmic) under registration No. COSM11935 and reported by IRAC database as to 8 cases, and is a somatic cell mutation in all the cases. The D49H mutation is not reported in the Human Genetic Mutation Database of Kyoto University targeting germ cell mutations, or Integrative Japanese Genome Mutation Database of the Tohoku University Tohoku Medical Megabank Organization. The D49H mutation has been reported by the Exome Aggregation Consortium (URL: http://exac.broadinstitute.org) database with an allele frequency of 0.000008261, whereas there is no report on the D49H mutation as to Li-Fraumeni syndrome, Li-Fraumeni like syndrome, or familial tumor which is caused by a germ cell mutation in p53. The D49H mutation has been reported by the dbSNP database (URL: http://www.ncbi.nlm.nih.gov/SNP/) under registration No. rs587780728, though the allele frequency of the Exome Aggregation Consortium is cited therein.

p53 works to prevent the wrong replication of unrepaired DNA by directly binding to homologous recombination related proteins, thereby inhibiting the works of these proteins, and suppressing the progression of homologous recombination. The D49H mutation in p53 is located in consecutive aspartic acid residues at positions 48 and 49 essential for the binding of the p53 protein to RPA (replication protein A) protein, which is a homologous recombination related protein. It has been reported that in cells caused to express p53 by simultaneously introducing both D48H and D49H mutations to both of these amino acid residues, RPA becomes able to function because no binding is formed between p53 and RPA; and as a result, homologous recombination is enhanced in a non-controlled state (non-patent document 1).

However, the document has studied using p53 in which both D48H and D49H mutations were simultaneously introduced, and has made no study on each amino acid residue alone at positions 48 and 49. Furthermore, the document has studied influence on the ability of D48H/D49H mutated p53 to bind to RPA and the ability to homologously recombine, but does not show the results of studying relation to the appearance of cancer or the extension of cancer, or information on clinical images of cases having the mutations. The introduction of the D48H/D49H mutations is absolutely based on the viewpoint of evaluating the functions of the sites.

**Prior Art Documents**

**Patent Document**

Patent document 1: Japanese unexamined Patent Application Publication No. 2003-265187

**Non-patent Document**

Non-patent document 1: Oncogene (2004) 23, 9025-9033

**Summary of the Invention**

**Object to be Solved by the Invention**

An object of the present invention is to provide a method for determining the presence or absence of a risk of developing a familial tumor which is caused by a germ cell mutation in p53.

**Means to Solve the Object**

The present inventors have prepared DNA samples from blood and cancer tissues of 1685 cancer patients and analyzed the DNA samples for the nucleotide sequences of exon regions using NGS. As a result, the present inventors have found that among the cancer patients, 6 patients had D49H mutation located on TAD2, which has been totally unknown so far in normal Japanese. The present inventors have also found one out of the 6 patients having D49H mutation has A159D mutation located on DBD. The present inventors have further confirmed that these mutations are germ cell mutations in all the cases. The present inventors have continued further data analysis and confirmed that one out of 795 cancer patients additionally investigated had D49H mutation, reaching the completion of the present invention.

As mentioned above, D49H mutation in p53 has been reported as very rare SNV of the germline of a healthy individual, but has not been reported as to cancer patients having a family history of cancer (patients suspected of familial cancer). In the present invention, D49H mutation in p53 has been found in 7 cancer patients having a family history of cancer. A germline mutation on TAD is very rare in familial cancer ascribable to a p53 mutation. From the family history or clinical information, it has been considered that the D49H mutation might become a factor for the appearance of cancer including Li-Fraumeni syndrome. Furthermore, A159D mutation in p53 is located on DBD for which a large number of mutations to deactivate the functions of p53 in tumor have been reported. Therefore, this mutation is similarly considered to become a factor for the appearance of cancer including Li-Fraumeni syndrome.

Specifically, the present invention is as follows.

(1) A method for determining the presence or absence of a risk of developing a familial tumor which is caused by a germ cell mutation in p53, comprising detecting the presence or absence of a mutation to substitute alanine with aspartic acid at codon 159 of human p53 and/or a mutation to substitute aspartic acid with histidine at codon 49 of human p53 in a biological sample collected from a test subject.

(2) The method according to (1), wherein the mutation to substitute alanine with aspartic acid is a mutation to substitute a nucleotide sequence "GCC" encoding alanine with a nucleotide sequence "GAC" encoding aspartic acid.

(3) The method according to (1), wherein the mutation to substitute aspartic acid with histidine is a mutation to substitute a nucleotide sequence "GAT" encoding aspartic acid with a nucleotide sequence "CAT" encoding histidine.

(4) The method according to (1) or (2), wherein the mutation to substitute alanine with aspartic acid is a germ cell mutation.

(5) The method according to (1) or (3), wherein the mutation to substitute aspartic acid with histidine is a germ cell mutation.

(6) The method according to any one of (1) to (5), wherein the presence or absence of the mutation is detected using a next generation DNA sequencer.

(7) The method according to any one of (1) to (5), wherein the presence or absence of the mutation is detected using a Sanger method.

**Effect of the Invention**

According to the present invention, the presence or absence of a risk of developing a familial tumor which is caused by a germ cell mutation in p53 can be determined on the basis of the presence or absence of A159D mutation and/or D49H mutation in a biological sample collected from a test subject. Particularly, when it has been determined that the risk of developing a familial tumor which is caused by a germ cell mutation in p53 is present, measures to prevent cancer, such as environmental improvement or lifestyle modification, can be taken on the basis of the results. Also, the development of cancer can be confirmed early by regular medical examination or the like. Furthermore, if a relative has developed a serious hereditary cancer-related disease such as Li-Fraumeni syndrome, presymptomatic diagnosis can be made on even a healthy person as to the possibility of developing the illness in the future.

**Brief Description of Drawings**

[Figure 1] Figure 1 shows a diagram visualizing the presence of a mutation in exon sequences of 6 cancer patients having D49H mutation using Integrative Genomics Viewer (IGV).

[Figure 2] Figure 2 is a diagram showing the sequencing (a portion) of human p53 gene by a Sanger method in 6 cancer patients having D49H mutation.

[Figure 3] Figure 3 is a schematic diagram of the constitution of six tandems of p53 transcriptional response element (TRE)-TATA box-firefly luciferase (Fire fly Luc) of Cignal p53 Reporter (luc) Kit (CCS-004L) from Qiagen N.V. used in luciferase assay.

[Figure 4] Figure 4 is a diagram showing results of the luciferase assay of each p53 mutation.

[Figure 5] Figure 5 is a diagram showing results of the immunohistochemical staining of each p53 mutation.

[Figure 6] Figure 6 is a diagram showing results of the immunoblotting of each p53 mutation.

[Figure 7] Figure 7(a) is a diagram showing the colony formation status after culture for 19 days of a line harboring each plasmid. Figure 7(b) is a graph showing the number of G418-resistant colonies formed after culture for 19 days of the line harboring each plasmid.

**Mode of Carrying Out the Invention**

The method for determining the presence or absence of a risk of developing a familial tumor which is caused by a germ cell mutation in p53 according to the present invention is not particularly limited as long as the method comprises detecting the presence or absence of A159D and/or D49H mutation in a biological sample collected from a test subject. Examples thereof can include a publicly known conventional sequencing method such as a Sanger method, and can preferably include a method of performing nucleotide sequencing using NGS because the determination can be made as to a large number of test subjects in a short time by one sequencing.

The nucleotide sequence of the human p53 gene can be represented as a sequence (SEQ ID NO: 1) indicated by the DNA sequence of the whole sequence of mature mRNA transcribed from the human p53 gene, for example, a nucleotide sequence consisting of 1182 bases from "start codon "aug"" to "stop codon "uga"" registered in Ensembl Transcript ID: ENST00000269305. This sequence of SEQ ID NO: 1 represents the nucleotide sequence of DNA of the wild type p53 gene without any mutation. The amino acid sequence of the wild type human p53 without any mutation can be represented as an amino acid sequence consisting of 393 amino acids represented by SEQ ID NO: 2.

Examples of the familial tumor which is caused by a germ cell mutation in p53 according to the present invention can include, but are not particularly limited to, malignant melanoma, skin cancer, lung cancer, trachea cancer, bronchial cancer, oral epithelial cancer, esophageal cancer, gastric carcinoma, colon cancer, rectum cancer, large intestine cancer, liver cancer, hepatocellular carcinoma, intrahepatic bile duct cancer, kidney cancer, pancreatic cancer, gastric carcinoma, prostate cancer, breast carcinoma, uterus cancer, ovary cancer, adenocarcinoma of the cecum, squamous cell carcinoma of the tongue, brain tumor, and osteosarcoma.

The test subject according to the present invention may be a cancer patient or may be a non-cancer patient.

Examples of the action of determining the presence or absence of a risk of developing a familial tumor which is caused by a germ cell mutation in p53 according to the present invention can include an action of determining that the test subject has already developed cancer or has a high risk of developing cancer when having D49H mutation, or that the test subject has a low risk of developing cancer caused by D49H mutation when having no D49H mutation. Examples of the D49H mutation can include a mutation to substitute bases "GAT" at positions 145 to 147 encoding aspartic acid with "CAT" or "CAC" encoding histidine in the wild type human p53 gene shown in SEQ ID NO: 1, and can preferably include a mutation to substitute the bases with "CAT".

Examples of the action of determining the presence or absence of a risk of developing a familial tumor which is caused by a germ cell mutation in p53 can also include an action of determining that the test subject has already developed cancer or has a high risk of developing cancer when having A159D mutation, or that the test subject has a low risk of developing cancer caused by A159D mutation when having no A159D mutation. Examples of the A159D mutation can include a mutation to substitute bases "GCC" at positions 475 to 477 encoding alanine with "GAC" or "GAT" encoding aspartic acid in the wild type human p53 gene shown in SEQ ID NO: 1, and can preferably include a mutation to substitute the bases with "GAC".

The method for determining the presence or absence of a risk of developing a familial tumor which is caused by a germ cell mutation in p53 according to the present invention includes an action of gathering data for the determination, but excludes diagnostic action by a physician. Moreover, overall determination can also be made by combining the results obtained by the method for determining the presence or absence of a risk of developing a familial tumor which is caused by a germ cell mutation in p53 according to the present invention with other examination results.

The biological sample collected from a test subject according to the present invention is not particularly limited as long as the sample enables detection of the presence or absence of D49H mutation in the test subject. Examples thereof can include an arbitrary biological sample generally used in nucleic acid collection, and can include a body fluid such as blood, plasma, serum, bone marrow fluid, semen, peritoneal fluid, urine, pleural effusion, pericardial fluid, and saliva, a tissue of hair, an organ, or the like, a cancer tissue and a cancer tissue lysate.

Examples of the blood sample can preferably include a buffy coat which is a layer of leukocyte and platelet formed between an erythrocyte layer and plasma by the centrifugation of blood, as a body fluid particularly useful for diagnosis. The buffy coat can be prepared by placing blood collected from the test subject in a blood collection tube, centrifuging the tube, and then recovering only a layer between an erythrocyte layer and plasma.

The cancer tissue lysate can be prepared by harvesting and cutting a cancer tissue of the test subject, and then lysing the cancer tissue with a proteolytic enzyme such as serine peptidase typified by protease K, followed by centrifugation to recover a supernatant.

Examples of the subject in which the presence or absence of A159D mutation and/or D49H mutation is to be confirmed can preferably include human p53 gene. Examples of the gene can preferably include genomic DNA and mRNA. The subject can be particularly preferably genomic DNA because RNA is less stable than DNA.

Examples of the method for preparing the genomic DNA can include a method of extracting the genomic DNA from the biological sample by a publicly known conventional method such as a method using phenol or chloroform. Preferably, highly pure DNA suitable for NGS can also be prepared using a commercially available kit such as DNeasy Blood & Tissue Kit or QIAamp DNA Blood Midi Kit (both manufactured by Qiagen N.V.).

The sequencing method using the NGS is a method based on a technique having the ability to sequence polynucleotides at an unprecedented speed and has the ability to process several hundreds to hundreds of millions of DNA fragments in parallel at large scale by reading a wide range of the genome by one sequencing using a plurality of primer sets. Each individual principle is used on a system basis as a sequencing mechanism. Examples thereof can preferably include each mechanism underlying a system from Illumina, Inc. using optical detection, Helicos True Single Molecule Sequencing (tSMS) system (see, for example, Harris T.D. et al., Science 320: 106-109 [2008]) which adopts a single molecule sequencing system, a system from Halcyon Molecular, Inc. using transmission electron microscopy (TEM), and a system using an ionic semiconductor sequencer from Life Technologies Corp. which performs sequencing through the use of the property of being capable of releasing ions when nucleotides are incorporated into a DNA strand. An ionic semiconductor sequencer which can efficiently amplify an enormous number of target regions through one PCR reaction using sets of approximately 294000 primer pairs was selected as the NGS according to the present invention.

In the case of performing sequencing using the NGS, the DNA sample needs to be prepared as a DNA library which is a mixture of DNA fragments having diverse lengths by fragmentation and is suitable for uniformly performing large-scale parallel sequencing. Each DNA fragment may be subjected to treatment such as addition of a fluorescent marker, beads, or the like, or ligation of an adaptor for specimen identification, for the sake of convenience of nucleotide sequence analysis.

Examples of the method for preparing the DNA library can include a publicly known conventional method. In the case of performing sequencing using NGS, it is preferred to use a DNA library production kit suitable for the model of each NGS. Examples thereof can include: a method using Ion plus fragment library kit, Ion PGM(TM) Sequencing 400 Kit, Ion AmpliSeq(TM) Library Kit 2.0, Ion PGM(TM) Template OT2 400 Kit, or the like for use of a NGS system from Life Technologies Corp.; and a method using GENSeq DNA Library Prep Kit or the like for use of a NGS system from Illumina, Inc. In the case of using any of these kits, the DNA library can be prepared according to the attached manual.

The prepared DNA library can also be prepared as a quantitative DNA library by quantification using an assay kit such as Qubit(R) assay kit.

The quantitative DNA library is preferably subjected to pretreatment prior to sequencing. Examples of the pretreatment prior to sequence analysis can include production of a chip for sequencing. Examples of the production of such a chip for sequencing can include a treatment of setting the library on a chip, then setting a reagent kit, a template solution, and the like necessary for sequencing, setting the chip on chip production equipment for sequencing, and then establishing running conditions, thereby automatically performing chip loading. Examples of the chip production equipment for sequencing can include Ion Chef(TM) system (manufactured by Life Technologies Corp.).

The DNA library thus pretreated for sequencing is automatically sequenced using NGS, for example, a system using the ionic semiconductor sequencer technique. The system exploits the character of releasing hydrogen ions as by-products when nucleotides are incorporated into a DNA strand by polymerase. Biochemical procedures of using a high-density array having an ion sensor, combining a semiconductor technique with simple sequencing chemistry, and chemically directly translating coded information (A, C, G, and T) into digital information (0 and 1) on a semiconductor chip are performed by a large-scale parallel method. For example, when a certain nucleotide is incorporated into a DNA strand, a hydrogen ion is released. Charge from this ion changes the pH of the solution. Therefore, a sequencer equipped with a solid-state pH meter is capable of sequencing by reading bases and converting chemical information to digital information. Examples of the nucleotide sequence that can be sequenced can include the whole genome sequence and the whole exon sequence. The whole exon sequence is preferred in terms of time efficiency. Examples of the model executing such a system can include Ion PGM(TM) system and Ion Proton(TM) system.

Alternatively, NGS may be performed using MiSeq system (manufactured by Illumina, Inc.), HiSeq system (manufactured by Illumina, Inc.), Genome Analyzer IIx (manufactured by Illumina, Inc.), Genome Sequencer-FLX (manufactured by F. Hoffmann-La Roche, Ltd.), or the like. The operation thereof can be performed according to the attached manual.

By the treatment with NGS, nucleotide sequence data obtained as raw data can be converted to specific base information in primary analysis (base calling) to obtain large quantities of data on nucleotide sequence fragments. In secondary analysis, these large quantities of data on nucleotide sequence fragments are converted to full-length sequence data by mapping to a reference genome sequence, and quality trimming such as removal of a duplicated sequence resulting from PCR, removal of an adaptor, removal of the 5' end or the 3' end, or removal of a location with consecutive sequences having low quality. These analyses can be conducted using software included in NGS usually used.

In tertiary analysis following the secondary analysis, output data of NGS is analyzed at a high speed to determine genetic properties, SNP, SNV, mutations, etc. of each individual person. Further, the sequence comparison analysis between blood-derived DNA samples and cancer-derived DNA samples using the determined sequence data can also be conducted to identify a mutation as a germ cell mutation or a somatic cell mutation. It can be determined that: when the blood-derived DNA sample has a mutation, the mutation is a germ cell mutation; and when the cancer-derived DNA sample has a mutation but the blood-derived DNA sample has no mutation, the mutation is a somatic cell mutation.

The sequencing can also be performed by further combination with panel analysis. The panel analysis according to the present invention can highly sensitively detect even a low-frequency mutation such as SNV by amplifying and sequencing a particular genome region such as an oncogene or a cancer-related gene. Examples thereof can specifically include analysis using Ion AmpliSeq(TM) Hotspot Panel (manufactured by Life Technologies Corp.) which provides 207 primer pairs designed targeting oncogenes and cancer suppressor genes as one tube of a primer pool and is suitable for exhaustively searching 2790 mutations, or Ion AmpliSeq(TM) Comprehensive Cancer Panel (manufactured by Life Technologies Corp.) which provides approximately 16000 primer pairs designed targeting oncogenes and cancer suppressor genes as 4 tubes of primer pools and enables comparison among specimens as to 409 cancer-related genes.

In addition, the presence or absence of A159D mutation and/or D49H mutation in human p53 in a biological sample collected from a test subject can be detected by using, alone or in combination, methods that can detect a single base substitution (point mutation) at a codon 159 site and/or a codon 49 site of human p53. Examples thereof can include a Sanger method which terminates DNA polymerase-mediated synthesis in a base-specific manner using dideoxynucleotides, and a pyrosequencing method exploiting the release of pyrophosphoric acid in association with the elongation reaction of DNA.

The primer for use in detecting a mutation at the codon 49 site of human p53 can be appropriately designed on the basis of sequence information on the human p53 gene and appropriately produced using an appropriate oligonucleotide synthesis apparatus. Examples of the primer of the Sanger method can include a forward primer: GCTGCCCTGGTAGGTTTTCT (SEQ ID NO: 3). The primer may contain one or more substitutions, deletions, or additions in the sequence thereof as long as the primer is capable of functioning as a primer for determining a sequence including the codon 49 site of the human p53 gene.

Likewise, examples of the primer for use in detecting a mutation at the codon 159 site of human p53 can include a forward primer: GTGAGGAATCAGAGGCCTGG (SEQ ID NO: 6). The primer may contain one or more substitutions, deletions, or additions in the sequence thereof as long as the primer is capable of functioning as a primer for determining a sequence including the codon 159 site of the human p53 gene.

The present invention is defined by the appended claims.

**Examples**

[Example 1]

[Whole exon analysis]

(Preparation of blood-derived DNA sample)

The blood of each of 1685 cancer patients was collected into 3 blood collection tubes (VENOJECT II vacuum blood collection tubes (sterilized products), manufactured by Terumo Corp., EDTA-2Na) and centrifuged at 4°C for 10 minutes in a refrigerated centrifuge (AX-320, manufactured by Tomy Seiko Co., Ltd.). Buffy coat parts from the 3 blood collection tubes were collected into one 15 mL centrifugal tube using a dropper to prepare a buffy coat fluid of each patient.

DNA was extracted from the buffy coat fluid of each patient mentioned above using QIAamp DNA Blood Midi Kit #51185 (manufactured by Qiagen N.V.). The extracted DNA solution was quantified using Qubit(R) assay kit. A 100 pM blood-derived DNA sample was prepared for each of the 1685 cancer patients.

(Preparation of cancer tissue-derived DNA sample)

Cancer tissues surgically harvested from the same 1685 cancer patients mentioned above were used as samples. Approximately 100 mg of the cancer tissues of each patient was chopped. In order to lyse the chopped cancer tissues, protease K was added thereto. The mixture was stirred at 54°C for 6 hours and then centrifuged at 4°C for 10 minutes in a refrigerated centrifuge (AX-320, manufactured by Tomy Seiko Co., Ltd.). Approximately 15 mL of the supernatant was placed in one centrifugal tube to prepare a cancer tissue lysate.

DNA was extracted from the cancer tissue lysate using QIAamp DNA Blood Midi Kit #51185. The extracted DNA solution was quantified using Qubit(R) assay kit. A 100 pM cancer tissue-derived DNA sample was prepared for each of the 1685 cancer patients.

(Preparation of DNA library)

The 100 pM blood-derived DNA sample and the 100 pM cancer tissue-derived DNA sample were each subjected (10 to 100 ng of DNA) to (1) amplification of a target region, (2) removal of a primer sequence, (3) ligation of a barcode adaptor for specimen identification, and (4) purification using Ion AmpliSeq(TM) Library kit 2.0 (manufactured by Thermo Fisher Scientific, Inc.). Subsequently, the sample was amplified using a thermal cycler and then purified to obtain a blood-derived DNA library and a cancer tissue-derived DNA library.

The blood-derived DNA library and the cancer tissue-derived DNA library were quantified by Q-PCR using Ion Library Quantitation kit (manufactured by Thermo Fisher Scientific, Inc.). The blood-derived DNA library and the cancer tissue-derived DNA library of the same patient were combined in equal amounts and diluted into 50 pM. 25 μL of the diluted libraries was set on an automatic pretreatment apparatus Ion Chef (manufactured by Thermo Fisher Scientific, Inc.) using a reaction reagent kit, Ion PI Hi-Q Chef kit, to obtain a chip for sequencing loaded with a template solution. The chip was mounted to Ion Proton (manufactured by Thermo Fisher Scientific, Inc.). Exon sequencing was performed using Ion PI Hi-Q Sequencing 200 kit (manufactured by Thermo Fisher Scientific, Inc.).

(Data analysis)

Raw data output by the sequencing was subjected to base calling using Torrent Suite software ver. 4.4 (manufactured by Thermo Fisher Scientific, Inc.), quality trimming, and mapping to a human reference sequence, UCSC hg19. 14 amplicons covering all 11 exons of the p53 gene were amplified, followed by the extraction of mutations different from the UCSC hg19 sequence using Torrent Variant Caller software (manufactured by Thermo Fisher Scientific, Inc.). Furthermore, a somatic cell-specific mutation was obtained by subtracting the "mutation different from the UCSC hg19 sequence in the blood-derived DNA" from the "mutation different from the UCSC hg19 sequence in the cancer tissue-derived DNA" of the same patient using Ion Reporter ver. 4.4 software (manufactured by Thermo Fisher Scientific, Inc.). By the method described above, blood-derived DNA mutation data, cancer tissue-derived DNA mutation data, and somatic cell-specific mutation data were obtained for the 1685 cancer patients.

(p53 gene mutation)

In order to search for specific mutations in the germlines of the cancer patients, the blood-derived DNA data of each of the 1685 patients was first compared with University of California, Santa Cruz (UCSC) genome browser (ver. hg19) [Kent WJ, Sugnet CW, Furey TS, Roskin KM, Pringle TH, Zahler AM and Haussler D (2002) The human genome browser at UCSC. Genome Res 12, 996-1006 (URL: https://genome.ucsc.edu/)], reportedly global human genome sequence standards, to extract 10 asynchronous substitutions that were germline gene polymorphisms or mutations in the p53 gene found in these subject patients and resulted in change in amino acid sequence. These polymorphisms or mutations included a gene polymorphism having no pathological significance, and a known or unknown pathological mutation responsible for hereditary cancer derived from p53 abnormality. Accordingly, these 10 substitutions were compared with the following public databases to speculate whether or not the substitutions would be known or unknown pathological germline mutations. (1) dbSNP [Sherry ST, Ward MH, Kholodov M, Baker J, Phan L, Smigielski EM and Sirotkin K (2001) dbSNP: the NCBI database of genetic variation. Nucleic Acids Res 29, 308-311 (URL: http://www.ncbi.nlm.nih.gov/SNP/)], (2) COSMIC [Forbes SA, Beare D, Gunasekaran P, Leung K, Bindal N, Boutselakis H, Ding M, Bamford S, Cole C, Ward S, Kok CY, Jia M, De T, Teague JW, Stratton MR, McDermott U and Campbell PJ (2015) COSMIC: exploring the world's knowledge of somatic mutations in human cancer. Nucleic Acids Res 43, D805-811(URL: http://cancer.sanger.ac.uk/cosmic)], (3) ClinVar [Landrum MJ, Lee JM, Benson M, Brown G, Chao C, Chitipiralla S, Gu B, Hart J, Hoffman D, Hoover J, Jang W, Katz K, Ovetsky M, Riley G, Sethi A, Tully R, Villamarin-Salomon R, Rubinstein W and Maglott DR (2016) ClinVar: public archive of interpretations of clinically relevant variants. Nucleic Acids Res 44, D862-868 (URL: http://www.ncbi.nlm.nih.gov/clinvar/)], (4) IARC TP53 database [Petitjean A, Mathe E, Kato S, Ishioka C, Tavtigian SV, Hainaut P and Olivier M (2007) Impact of mutant p53 functional properties on TP53 mutation patterns and tumor phenotype: lessons from recent developments in the IARC TP53 database. Hum Mutat 28, 622-629 (URL: http://p53.iarc.fr/)], (5) iJGVD. [Nagasaki M, Yasuda J, Katsuoka F, Nariai N, Kojima K, Kawai Y, Yamaguchi-Kabata Y, Yokozawa J, Danjoh I, Saito S, Sato Y, Mimori T, Tsuda K, Saito R, Pan X, Nishikawa S, Ito S, Kuroki Y, Tanabe O, Fuse N, Kuriyama S, Kiyomoto H, Hozawa A, Minegishi N, Douglas Engel J, Kinoshita K, Kure S, Yaegashi N, ToMMo Japanese Reference Panel Project and Yamamoto M (2015) Rare variant discovery by deep whole-genome sequencing of 1,070 Japanese individuals. Nat Commun 6, 8018 (URL: http://ijgvd.megabank.tohoku.ac.jp/). (6) HGVD [Higasa K, Miyake N, Yoshimura J, Okamura K, Niihori T, Saitsu H, Doi K, Shimizu M, Nakabayashi K, Aoki Y, Tsurusaki Y, Morishita S, Kawaguchi T, Migita O, Nakayama K, Nakashima M, Mitsui J, Narahara M, Hayashi K, Funayama R, Yamaguchi D, Ishiura H, Ko WY, Hata K, Nagashima T, Yamada R, Matsubara Y, Umezawa A, Tsuji S, Matsumoto N and Matsuda F (2016) Human genetic variation database, a reference database of genetic variations in the Japanese population. J Hum Genet (2016) doi:101038/jhg201612 (URL: http://www.genome.med.kyoto-u.ac.jp/SnpDB/)], (7)ExAC [Exome Aggregation Consortium (ExAC) (URL: http://exac.broadinstitute.org) (Version 0.3.1).]

As a result, 3 of the 10 types was excluded from this invention because reported as gene polymorphisms or already reported as causative gene mutations of Li-Fraumeni syndrome-related hereditary cancer. Furthermore, the public database (4) IARC TP53 database describes the functions of variant proteins based on *in vitro* experiments. Therefore, the presence of mutations was established by the Sanger method or the like by focusing attention on 3 mutations, D49H, Q144R, and A159D, found to cause functional abnormality among the remaining 7 types. In addition, the D49H mutation was considered to be a probable causative gene from information showing that: this mutation was found in 6 out of 1685 cases (0.36%); all the cases had a family history of cancer; one of the cases had Li-Fraumeni like syndrome; etc. The A159D mutation was considered to have pathological significance because this mutation coexisted with the D49H mutation and was found in cases manifesting Li-Fraumeni like syndrome.

As for the D49H mutation, among the above public databases, (2) COSMIC and (4) the IARC TP53 database have reported 8 cases with a somatic cell mutation that was not a germline mutation and appeared in cancer for the first time. Also from this finding, involvement thereof in cell carcinogenesis is speculated.

Whether or not a polymorphism or a mutation in a certain gene has pathological significance is determined by using, as a useful index, the fact that a gene polymorphism found with a relatively high frequency (1% or more) in healthy persons is less likely to have pathological significance, whereas a mutation that is of less than 1% and is very rare or, furthermore, is found at a high rate in cancer is likely to have pathological significance. It has been merely reported as to the D49H mutation that: the D49H mutation was found with a high frequency of 0.36% in cancer patients from the results of this test; all the patients having the D49H mutation had a family history of cancer; furthermore, there is no report on this mutation (0.1% or less) in (5) iJGVD or (6)HGVD targeting Japanese among the public databases targeting germline mutations; and the D49H mutation exhibits an appearance frequency as very low as 0.0008% in terms of allele frequency only in the public database (7) ExAC, and the relation thereof to a disease is not shown.

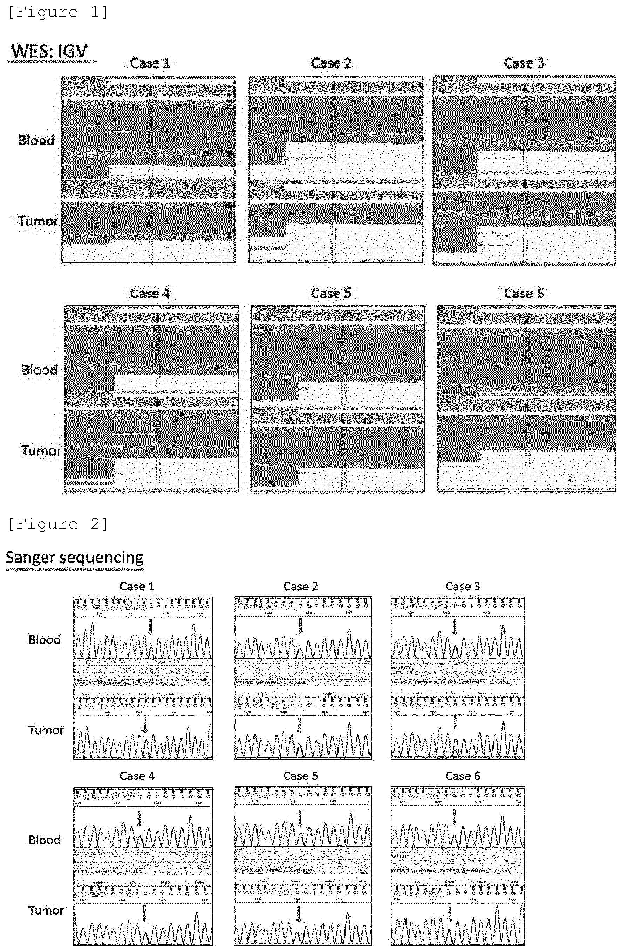

The presence of mutations in 6 persons was visualized using visualization software for next generation DNA sequencing (Integrative Genomics Viewer) (J.T. Robinson, H. Thorvaldsdottir, W. Winckler et al., Integrative genomics viewer, Nat. Biotechnol. 29 (2011) 24-26) . The results are shown as Integrative Genomics Viewer (IGV) in Figure 1. All the 6 persons were confirmed to have D49H mutation in p53.

The presence of the D49H mutation obtained in this blood-derived DNA data was confirmed as to the cancer tissue-derived DNA mutation data. As a result, the D49H mutation was present in 6 of the 1685 persons.

(Panel analysis)

For further confirmation, panel analysis was conducted on the cancer tissue-derived DNA to exhaustively analyze mutations on subject genes with the coding regions of 409 human cancer-related genes as target regions.

Mutations on subject genes were exhaustively analyzed from 10 ng of the cancer tissue DNA with the coding regions of 409 human cancer-related genes as target regions using Ion AmpliSeq(TM) Library Kit 2.0 and Ion AmpliSeq Comprehensive Cancer Panel (both manufactured by Thermo Fisher Scientific, Inc.). After amplification of the target regions, removal of a primer sequence, and ligation of a barcode adaptor for specimen identification, the sample was amplified using a thermal cycler and purified to obtain a cancer tissue-derived DNA library.

The cancer tissue-derived DNA library was quantified by Q-PCR using Ion Library Quantitation Kit (manufactured by Thermo Fisher Scientific, Inc.) and diluted into 100 pM. 8 µL of the diluted library was set on an automatic pretreatment apparatus Ion Chef (manufactured by Thermo Fisher Scientific, Inc.) using a reaction reagent kit, Ion PI Hi-Q OT2 200 Kit (manufactured by Thermo Fisher Scientific, Inc.) to obtain a chip for sequencing loaded with a template solution. The chip was mounted to Ion Proton (manufactured by Thermo Fisher Scientific, Inc.). Sequencing was performed using Ion PI Hi-Q Sequencing 200 kit (manufactured by Thermo Fisher Scientific, Inc.).

(Data analysis)

Output raw data obtained by the sequencing was subjected to base calling using Torrent Suite software ver. 4.4 (manufactured by Thermo Fisher Scientific, Inc.), quality trimming, and mapping to reference sequences of the 409 targeted cancer-related genes in a human reference sequence, Comprehensive Cancer Panel to extract mutations.

The 409 targeted cancer-related genes are shown below.

PDE4DIP IL7R MMP2 IRF4 AURKB TLR4 ERBB4 PAX7 FH SBDS MTOR ERCC5 SDHC LPP CKS1B ATRX TCF3 BLNK UBR5 FOXP4 SUFU NBN WT1 MPL KDR TP53 NTRK1 PTEN CDK6 AFF1 TCF7L1 PDGFRB REL MLL3 EP300 MALT1 MITF HNF1A EPHA3 SETD2 STK11 FLT1 TGFBR2 LCK MEN1 NUP98 FANCA RAF1 RARA ERG EXT1 TRIM33 NLRP1 MARK4 MYD88 SMAD2 NSD1 PML MAP3K7 FGFR1 ERCC1 MET BAI3 NUP214 BLM AXL CREBBP SDHB ZNF384 GATA3 MN1 MAP2K2 SH2D1A PTPRD UGT1A1 SGK1 TCF7L2 PDGFB PIK3C2B TFE3 JAK3 JAK1 CSMD3 FGFR2 FLCN ITGA9 ERCC4 KIT PRKAR1A EXT2 NCOA2 GDNF CTNNA1 CYP2D6 EPHA7 STK36 GATA2 HOOK3 ABL2 CCND2 AKT1 LAMP1 JAK2 PIK3R1 GRM8 CDKN2C CYP2C19 MAF SMO PDGFRA BMPR1A TIMP3 ETV4 TGM7 TET1 MAPK1 FOXP1 IGF2 LRP1B MAML2 EPHB6 TSC1 COL1A1 TLX1 ASXL1 MYH11 BRIP1 AURKC TNK2 IDH1 PTPN11 KDM5C PAX3 PER1 CARD11 PTGS2 SDHD POU5F1 NF1 BIRC5 PALB2 MLL2 SEPT9 CDK12 PIK3CG WHSC1 GNAS PLCG1 MDM2 DDB2 MLL GUCY1A2 CTNNB1 AKT2 MYCL1 MAP2K1 EGFR NFKB1 PAK3 IKBKB IL21R BCL3 MTRR LIFR DICER1 SOX11 CBL ITGB3 IKZF1 DNMT3A TCL1A CCNE1 TBX22 TAF1 CIC CD79A BCR CCND1 RUNX1T1 SMUG1 PIK3CA IGF2R NOTCH2 FLT3 PRKDC ALK EZH2 PSIP1 MSH6 WAS PMS1 IL2 ERBB3 ATM CDK8 FAS TAF1L BCL11A NUMA1 ESR1 ERBB2 CREB1 NIN SAMD9 SYK ARNT CASC5 DDIT3 MARK1 ADAMTS20 CDK4 CDH1 EP400 DDR2 PLAG1 CD79B DEK FLI1 CRTC1 IRS2 SMARCB1 CMPK1 DCC CHEK1 SMARCA4 XPO1 FOXL2 LTK MUC1 GNAQ BCL2 NFKB2 MLH1 XPA HRAS EML4 PTPRT RALGDS PIK3CB FOXO3 MYH9 MAP2K4 ITGB2 PPP2R1A TET2 ING4 IDH2 APC SMAD4 BCL6 CDKN2B NPM1 FGFR4 G6PD AKAP9 CDH11 PIK3CD CDKN2A BCL9 MAPK8 ERCC3 PTCH1 RECQL4 IGF1R TPR BCL10 BRD3 PGAP3 SF3B1 TSHR MYC KAT6A THBS1 RHOH ATR GNA11 TAL1 JUN CSF1R ETV1 BCL2L1 BCL11B RNASEL BIRC2 NTRK3 PIK3R2 ABL1 KEAP1 PLEKHG5 NF2 CRBN DPYD GPR124 SSX1 TSC2 FANCF AR CRKL CDH2 DAXX KDM6A FLT4 ATF1 IL6ST LTF FGFR3 HSP90AB1 NKX2-1 MAGI1 ETS1 TCF12 PAD50 ARID2 KRAS BCL2L2 MYCN SYNE1 BRAF PAX5 NCOA1 NOTCH1 PPARG AKT3 TNFAIP3 NCOA4 CHEK2 CDH5 FOXO1 PKHD1 MCL1 MUTYH FANCC PAX8 IKBKE HIF1A TRRAP SOCS1 CDH20 EPHB4 ZNF521 HLF RET RUNX1 XPC ARID1A MRE11A MBD1 TNFRSF14 HCAR1 EPHB1 RB1 CDC73 KAT6B SOX2 FAM123B SDHA NRAS AURKA LPHN3 VHL WRN DST BAP1 ROS1 MSH2 CYLD SRC FBXW7 MDM4 CEBPA GATA1 ERCC2 PBX1 PRDM1 RPS6KA2 FN1 MTR BUB1B PHOX2B PBRM1 FANCG HSP90AA1 ICK MLLT10 RRM1 MAGEA1 FANCD2 PIM1 TRIM24 USP9X TRIP11 MAFB NFE2L2 PMS2 RNF2 NOTCH4 KLF6 BIRC3 RNF213 PARP1 ACVR2A TOP1 POT1 AFF3 MYB FZR1 XRCC2 BTK ITGA10

(Results)

As a result of panel analysis, the same 6 cancer patients as above were confirmed to have D49H mutation.

The details of these 6 persons are described in Table 1 below. The cancer types of the 6 persons are osteosarcoma, breast carcinoma, squamous cell carcinoma of the tongue, adenocarcinoma of the cecum, hepatocellular carcinoma, and gastric carcinoma, respectively. The D49H mutation was heterozygous in all the cases. Also, all of these 6 persons were cancer patients having a family history. The 12-year-old boy with osteosarcoma of patient No. 1 had not only D49H mutation but a mutation to substitute alanine with aspartic acid at codon 159 (A159D) in the p53 gene. In addition to the 6 persons shown in Table 1, one out of 795 cancer patients additionally analyzed was further confirmed to have D49H mutation.

In Li-Fraumeni syndrome, Li-Fraumeni like syndrome, and familial tumor caused by a germ cell mutation in p53, the position of the mutation is focused on DBD, and there is no previous report on D49H mutation. There is no report on A159D as to Li-Fraumeni syndrome, Li-Fraumeni like syndrome, or familial tumor caused by a germ cell mutation in p53.

[Table 1]

| Case No. | Age | Sex | Pathological diagnosis | Past history of cancer (Age) | Germline mutation | Heterozygosity in blood cells | Somatic mutaions of cancer driver genes detected in tumor tissues | Family history (Age) |
|---|---|---|---|---|---|---|---|---|
| 1 | 12 | M | Osteosarcoma | None | D49H A159D | Heterozygous | None | (1st, mother) Lung ca (37) & Ovarian ca (37) (2nd, grandmather) Breast ca (NOS) |
| 2 | 43 | F | Breast ca | None | D49H | Heterozygous | *PTEN, BRCA1* | (2nd, aunt) Breast ca (late 40s) (2nd, grandmother) Gastric ca (NOS) |
| 3 | 60 | F | Sq cell ca of the tongue | None | D49H | Heterozygous | *TP53, CASP8* | (1st, father) Prostate ca (NOS) |
| 4 | 62 | F | Adenoca of the cecum | None | D49H | Heterozygous | *KRAS, PIK3CA, CREBBP* | (1st, mother) Breast ca (50s) (1st, brother) Lung ca (50s) |
| 5 | 63 | M | Hepatocellular ca | Rectal ca (49) | D49H | Heterozygous | *KDM6A, U2AF1* | (2nd, uncle) Cancer (NOS) |
| 6 | 79 | M | Gastric ca | None | D49H | Heterozygous | *APC, NOTCHI, CBL, KMT2D, PTPN11 B2M, MAP2KI, CDH1, NCOR1, BRCA1 DNMT1, SMARCA4, GNAS* | (1st, father) Gastric ca (NOS) |

(Sanger method)

The D49H mutation was confirmed by sequencing according to the Sanger method. The primer sequences used are as follows.

Forward primer: GCTGCCCTGGTAGGTTTTCT (SEQ ID NO: 3)
Reverse primer: GTGGATCCATTGGAAGGGCA (SEQ ID NO: 4)

The PCR reaction mixture is as follows.

| PCR reaction mixture | Final concentration |
| --- | --- |
| HotStarTaq Master Mix, 2× | 12.5 μL |
| Forward primer (10 μM) 0.5 μl | 0.2 μM |
| Reverse primer (10 μM) 0.5 μl | 0.2 μM |
| RNase-free water | 10.5 μL |
| Template DNA (50 ng/μl) | 1.0 μL |
| Total volume | 25.0 μL |

The thermal cycler conditions are as follows.

| Initial PCR activation step | 15 min, 95°C |
| --- | --- |
| 3-step cycling: | |
| Denaturation | 1 min, 94°C |
| Annealing | 1 min, 60°C |
| Extension | 1 min, 72°C |
| Number of cycles | 35 cycles |
| Final extension | 10 min, 72°C |

The sequence analysis of the obtained amplicon (amplification product) was consigned to Takara Bio Inc. The primer for Sanger sequencing used was a forward primer: GCTGCCCTGGTAGGTTTTCT (SEQ ID NO: 3). As a result, all the 6 cases were confirmed to have a mutation from gat to Cat at the position of codon 49.

The sequence of the amplicon was the sequence represented by SEQ ID NO: 5 in all the 6 persons of patient Nos. 1 to 6 shown in Table 1. The results described above were also similarly applicable to the additionally determined 1 patient having D49H mutation.

```
GTGGATCCATTGGAAGGGCAggcccaccacccccaccccaaccccagccccctagcag

agacctgtgggaagcgaaaattccatgggactgactttctgctcttgtctttcagact

tcctgaaaacaacgttctggtaaggacaagggttgggctggggacctggagggctggg

gacctggagggctggggggctggggggctgaggacctggtccctgactgctcttttca

cccatctacagtccccttgccgtcccaagcaatggatgatttgatgctgtccccgga

cCatattgaacaatggttcactgaagacccaggtccagatgaagctcccagaatgcca

gaggctgctcccccgtggcccctgcaccagcagctcctacaccggcggccctgcac

cagccccctcctggcccctgtcatcttctgtcccttcccAGAAAACCTACCAGGGCAG

C (SEQ ID NO: 5)
```

Sequencing was performed as to A159D detected in patient No. 1. The primer sequence used is as follows.

Forward primer: GTGAGGAATCAGAGGCCTGG (SEQ ID NO: 6)
Reverse primer: GCACACCTATAGTCCCAGCC (SEQ ID NO: 7)

The PCR reaction mixture is as follows.

| PCR reaction mixture | Final concentration |
| --- | --- |
| HotStarTaq Master Mix, 2× | 12.5 μL |
| Forward primer (10 μM) 0.5 μl | 0.2 μM |
| Reverse primer (10 μM) 0.5 μl | 0.2 μM |
| RNase-free water | 10.5 μL |
| Template DNA (50 ng/μl) | 1.0 μL |
| Total volume | 25.0 μL |

Thermal cycler conditions are as follows.

| Initial PCR activation step | 15 min, 95°C |
| --- | --- |
| 3-step cycling: | |
| Denaturation | 1 min, 94°C |
| Annealing | 1 min, 60°C |
| Extension | 1 min, 72°C |
| Number of cycles | 35 cycles |
| Final extension | 10 min, 72°C |

The sequence analysis of the obtained amplicon (amplification product) was consigned to Takara Bio Inc. The primer for Sanger sequencing used was a forward primer: GTGAGGAATCAGAGGCCTGG (SEQ ID NO: 6). As a result, a mutation from gcc to gAc was detected as shown in SEQ ID NO: 8 below. This corresponds to a substitution of alanine with aspartic acid at the position of codon 159.

GCACACCTATAGTCCCAGCCacttaggaggctgaggtgggaagatcacttgaggccag

gagatggaggctgcagtgagctgtgatcacaccactgtgctccagcctgagtgacaga

gcaagaccctatctcaaaaaaaaaaaaaaaaagaaaagctcctgaggtgtagacgcc

aactctctctagctcgctagtgggttgcaggaggtgcttacgcatgtttgtttctttg

ctgccgtcttccagttgctttatctgttcacttgtgccctgactttcaactctgtctc

cttcctcttcctacagtactccctgccctcaacaagatgttttgccaactggccaag

acctgccctgtgcagctgtgggttgattccacaccccgcccggcacccgcgtccgcg

Acatggccatctacaagcagtcacagcacatgacggaggttgtgaggcgctgccccca

ccatgagcgctgctcagatagcgatggtgagcagctggggctggagagacgacagggc

tggttgcccagggtccccaggCCTCTGATTCCTCAC  (SEQ ID NO: 8)

[Example 2]

[Transcriptional activity of mutated p53 gene]

(Production of mutated p53 gene expression plasmid)

How the D49H and A159D mutations each influenced the transcriptional activity of p53 was studied by conducting a reporter assay using a Saos-2 cell line, a p53 null osteosarcoma cell line lacking p53. Plasmids capable of causing expression of 6 types of mutated p53 shown below in cultured cells were produced and used in the study. Each plasmid contained a wild type p53 sequence consisting of 1182 bases, and was produced through PCR reaction using EX-B0105-M02 plasmid (manufactured by GeneCopoeia, Inc.) capable of causing expression of wild type p53 in a human cytomegalovirus (CMV) promoter-dependent manner as a template, PrimeSTAR(R) Mutagenesis Basal kit (manufactured by Takara Bio Inc.), and the primers shown in Table 2 below. Then, the PCR reaction product was transferred to transformation competent *E. coli* HST08 Premium Competent Cells (manufactured by Takara Bio Inc.). *E. coli* colonies were isolated in an ampicillin-containing LB agar culture medium and thereby cloned. Each clone was cultured in an ampicillin-containing LB culture medium and recovered, followed by the extraction of a plasmid using QIAprep Spin Miniprep Kit (manufactured by Qiagen N.V.). The introduction of the mutations of interest was confirmed by the Sanger method using the plasmid.

[Table 2]

| Mutation | Mutation introduction position | Primer name | Sequence (5'-3') lower-case letter represents mutation-introduced base |
|---|---|---|---|
| D49H | c.145G>C | TP53_D49H-sdmF2 | CCCGGACcATATTGAACAATGGTTCA (SEQ ID NO: 9) |
| | | TP53_D49H-sdmR2 | TCAATATgGTCCGGGGACAGCATCAA (SEQ ID NO: 10) |
| A159D | c.476C>A | TP53_A159D-sdmF2 | GTCCGCGaCATGGCCATCTACAAGCA (SEQ ID NO: 11) |
| | | TP53_A159D-sdmR2 | GGCCATGtCGCGGACGCGGGTGCCGG (SEQ ID NO: 12) |
| S46A | c.136T>G | TP53_S46A-sdmF2 | GATGCTGgCCCCGGACGATATTGAAC (SEQ ID NO: 13) |
| | | TP53_S46A-sdmR2 | TCCGGGGcCAGCATCAAATCATCCAT (SEQ ID NO: 14) |
| P47S | c.139C>T | TP63_P47S-sdmF2 | GCTGTCCtCGGACGATATTGAACAAT (SEQ ID NO: 15) |
| | | TP53_P47S-sdmR2 | TCGTCCGaGGACAGCATCAAATCATC (SEQ ID NO: 16) |
| D49A | c.146A>C | TP53_D49A-sdmF2 | CCGGACGcTATTGAACAATGGTTCAC (SEQ ID NO: 17) |
| | | TP53_D49A-sdmR2 | TTCAATAgCGTCCGGGGACAGCATCA (SEQ ID NO: 18) |
| R175H | c.524G>A | TP53_R175H-sdmF2 | GTGAGGCaCTGCCCCCACCATGAGCG (SEQ ID NO: 19) |
| | | TP53_R175H-sdmR2 | GGGGCAGtGCCTCACAACCTCCGTCA (SEQ ID NO: 20) |

The PCR reaction mixture is as follows.

PrimeSTAR Max Premix, 2×: 5.0 μL

Forward primer (5 pmol/μL): 0.5 μL

Reverse primer (5 pmol/μL): 0.5 μL

DNase-free water: 4.0 μL

EX-B0105-M02 plasmid (20 pg/μL): 1.0 μL

Total volume: 11.0 μL

Thermal cycler conditions are as follows.

98°C for 20 sec

98°C for 10 sec*

55°C for 20 sec*

72°C for 2 min*

*40 cycles

72°C for 2 min

The sequence analysis of the plasmids was consigned to Eurofins Genomics K.K. The primers for Sanger sequencing used were pEZ-M02-SeqF: CAGCCTCCGGACTCTAGC (SEQ ID NO: 21), pEZ-M02-SeqR: TAATACGACTCACTATAGGG (SEQ ID NO: 22), and TP53-SR3: GAGGAGCTGGTGTTGTTG (SEQ ID NO: 23).

(Mutated p53 gene)

The 6 types of mutated p53 genes used in this study are as described below. 4 types of mutations other than D49H and A159D were used as experimental controls.

1) D49H: a mutation present on TAD2 of the wild type human p53 gene. The mutation substitutes bases "GAT" at positions 145 to 147 encoding aspartic acid in the sequence represented by SEQ ID NO: 1 by "CAT" encoding histidine.
2) A159D: a mutation present on DBD of the wild type human p53 gene. The mutation substitutes bases "GCC" at positions 475 to 477 encoding alanine in the sequence represented by SEQ ID NO: 1 by "GAC" encoding aspartic acid.
3) S46A: a mutation present on TAD2 of the wild type human p53 gene. The mutation substitutes bases "TCC" at positions 136 to 138 encoding serine in the sequence represented by SEQ ID NO: 1 by "GCC" encoding alanine. The phosphorylation of this site is important for the induction of apoptosis.
4) P47S: a mutation present on TAD2 of the wild type human p53 gene. The mutation substitutes bases "CCG" at positions 139 to 141 encoding proline in the sequence represented by SEQ ID NO: 1 by "TCG" encoding serine. It is known that activated p53 becomes more sensitive to intracellular oxidative stress by suppressing the expression of a cystine/glutamate exchanger (xCT), and induces cell death (ferroptosis) in the presence of oxidative stress. P47S is known to reduce only the ability of p53 to induce ferroptosis.
5) D49A: a mutation present on TAD2 of the wild type human p53 gene. The mutation substitutes bases "GAT" at positions 145 to 147 encoding aspartic acid in the sequence represented by SEQ ID NO: 1 by "GCT" encoding alanine. This mutation is a mutation that influences the interaction between a CREB binding protein binding to p53 and a NCBD domain.
6) R175H: a mutation present on DBD of the wild type human p53 gene. The mutation substitutes bases "CGC" at positions 523 to 525 encoding arginine in the sequence represented by SEQ ID NO: 1 by "CAC" encoding histidine. This mutation is a mutation present on DBD that is detected with a very high frequency in tumor.

(Transfection for reporter assay aimed at measuring transcriptional activity of mutated p53 gene)

In this experiment, a total of 8 types of plasmids for gene expression were used: an experimental negative control plasmid pReceiver-M02CT (vacant plasmid free from the p53 sequence, manufactured by GeneCopoeia, Inc.),

a wild type p53 expression plasmid (EX-B0105-M02 plasmid) and the produced 6 types of mutated p53 expression plasmids. Two types of plasmid mixed solutions, i.e., a plasmid mixed solution of 500 ng of each gene expression plasmid described above and 2.5 $\mu$L of p53 reporter mix (containing p53 TRE-TATA box-firefly luciferase plasmid and Renilla luciferase, manufactured by Qiagen N.V.), and a plasmid mixed solution of 2.5 $\mu$L of negative control mix (containing a vacant firefly luciferase reporter plasmid free from the p53TRE sequence, and Renilla luciferase, manufactured by Qiagen N.V.) were provided as to each of the 8 types of gene expression plasmids. To each of a total of 16 types of plasmid mixed solutions, 1.5 $\mu$L of P3000 solution (manufactured by Thermo Fisher Scientific, Inc.) was added, and Opti-MEM culture medium (manufactured by Thermo Fisher Scientific, Inc.) was added to adjust the total volume to 25 $\mu$L. Then, the mixture was left standing at room temperature for 30 minutes. For each plasmid mixed solution, 1.5 $\mu$L of Lipofectamine 3000 solution (manufactured by Thermo Fisher Scientific, Inc.) was mixed with 23.5 $\mu$L of Opti-MEM culture medium to provide a Lipofectamine mixed solution, which was then added to each plasmid mixed solution described above and gradually mixed therewith. Then, the mixture was left standing at room temperature for 10 minutes to prepare a transfection solution. Transfection treatment was performed by adding dropwise this whole amount, 50 $\mu$L, of each transfection solution to 1 well of Saos-2 cells cultured for 24 hours using Roswell Park Memorial Institute culture medium 1640 (RPMI1640, Thermo Fisher Scientific, Inc.) containing 10% (v/v) heat inactivated fetal bovine serum on a 24-well plate (Corning Inc.). The cells used in this transfection were provided by dispensing, on the day therebefore, a cell suspension of $8 \times 10^4$ Saos-2 cells suspended in 0.5 mL of RPMI1640 culture medium containing 10% (v/v) heat inactivated fetal bovine serum to each well of a 24-well plate.

(Cell culture)

After the Lipofection treatment, the cells were cultured for 24 hours. Culture medium replacement was performed for the removal of the transfection solution. The cells were further cultured for 24 hours. Each cell thus cultured was used as a cell sample for reporter assay using Dual-Glo Luciferase Reporter System (manufactured by Promega Corp.) capable of quantifying 2 types of luciferase activities as stable luminescent signals in cells.

(Luciferase assay)

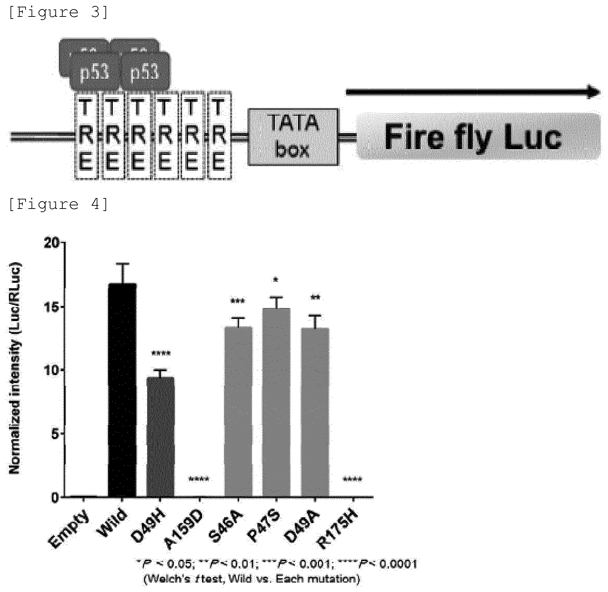

After removal of the culture medium, 100 $\mu$L of Reporter Lysis Buffer (manufactured by Promega Corp.) was added to each well. The cells were lysed by repeating freezing and thawing three times. 80 $\mu$L of the cell lysate was transferred to a 96-well PCR plate (manufactured by Nippon Genetics Co., Ltd.) and centrifuged at 460 g for 2 minutes to precipitate residues resulting from the cell lysis. 50 $\mu$L of the supernatant was transferred to a 96-well plate for chemiluminescence measurement (manufactured by Corning Inc.), and thereto 50 $\mu$L of Dual-Glo Luciferase reagent (manufactured by Promega Corp.) was added. The plate was agitated at room temperature for 15 minutes using a shaker, followed by the measurement of the luminescence value of firefly luciferase using GLOMAX multi detection system (manufactured by Promega Corp.). Subsequently, 50 $\mu$L of Dual-Glo Stop & Glo Reagent (manufactured by Promega Corp.) was added. The plate was agitated at room temperature for 15 minutes using a shaker to perform the quenching of firefly luciferase and the luminescence reaction of Renilla luciferase, followed by the measurement of the luminescence value thereof using GLOMAX multi detection system. The results are shown in Figure 4. The Y axis (Luc/RLuc) of Figure 4 depicts a fluorescence value as transcriptional activity, wherein the fluorescence value was standardized by dividing firefly luciferase activity (Luc) obtained in the presence of the Saos-2 cells harboring each plasmid by the results about Renilla luciferase (RLuc) as an internal standard control value for data.

(Results)

As is evident from Figure 4, the transcriptional activity of D49H mutated p53 was lower by approximately 45% than the transcriptional activity of wild type p53. On the other hand, the transcriptional activity of A159D mutated p53 was rarely detected.

R175H mutation in p53 which is detected with a very high frequency in malignant tumor is known to completely inactivate the functions of p53 by influencing the conformational stability of p53 (Oncogene (2007) 26, 2226-2242, etc.). The transcriptional activity was shown to disappear completely in a Saos-2 cell line in which this R175H mutation was introduced. The A159D mutated p53 also exhibited a marked decrease in transcriptional activity at the same level as in decrease in the transcriptional activity of R175H mutated p53.

The transcriptional activity of S46A mutated p53 was lower by approximately 20% than that of wild type p53. The transcriptional activity of P47S mutated p53 was lower by approximately 12% than that of wild type p53. The transcriptional activity of D49A mutated p53 was lower by approximately 20% than that of wild type p53, confirming a slight decrease in transcriptional activity in cells having a mutation on TAD2 other than D49H.

The study of this Example 2 on the influence of each mutation on the transcriptional activity of p53 was conducted by a similar experimental method using lung cancer-derived NIH-H1299 cells of a p53 null cell line, as with the Saos-2 cells. The disappearance of transcriptional activity was observed in A159D mutated p53. Approximately 40% decrease in the transcriptional activity was observed in D49H mutated p53 compared with wild type p53. In transcriptional activity measurement using both the p53 null cell lines, D49H mutated p53 and A159D mutated p53 exhibited results in agreement (data not shown).

[Example 3]

[Study on nuclear translocation]

In order to confirm whether or not the decreased transcriptional activity of D49H mutated p53 and A159D mutated p53 was caused by a decrease in the frequency of nuclear translocation, a p53 null Saos-2 cell line was caused to transiently express A159D mutated p53, D49H mutated p53, and wild type p53, and the intracellular localization of each p53 protein was studied by immunohistochemical staining. A plasmid pReceiver-M02CT was used as a negative control.

A cell suspension of $8 \times 10^4$ Saos-2 cells suspended in 0.5 mL of RPMI1640 culture medium containing 10% (v/v) heat inactivated fetal bovine serum was dispensed to each well of 4-well Nunc Lab-Tek Chamber Slide System (manufactured by Thermo Fisher Scientific, Inc.) and cultured for 24 hours. To 500 ng of each DNA solution of each mutated p53 expression plasmid described above, a wild type p53 expression plasmid, or pReceiver-M02CT plasmid, 1 μL of P3000 solution (manufactured by Thermo Fisher Scientific, Inc.) was added, and Opti-MEM culture medium (manufactured by Thermo Fisher Scientific, Inc.) was added to adjust the total volume to 25 μL. Then, the mixture was left standing at room temperature for 30 minutes. For each plasmid solution thus prepared, 1.5 μL of Lipofectamine 3000 solution (manufactured by Thermo Fisher Scientific, Inc.) was mixed with 23.5 μL of Opti-MEM culture medium to provide a Lipofectamine mixed solution, which was then added to each plasmid solution described above and gradually mixed therewith. Then, the mixture was left standing at room temperature for 10 minutes to prepare a transfection solution. Transfection treatment was performed by adding dropwise this whole amount, 50 μL, of each transfection solution to 1 well of the Saos-2 cells cultured on 4-well Nunc Lab-Tek Chamber Slide System from the day therebefore. Then, the cells were cultured for 24 hours. Culture medium replacement was performed for the removal of the transfection solution. Then, the cells fixed by further culture for 24 hours were subjected to immunohistochemical staining.

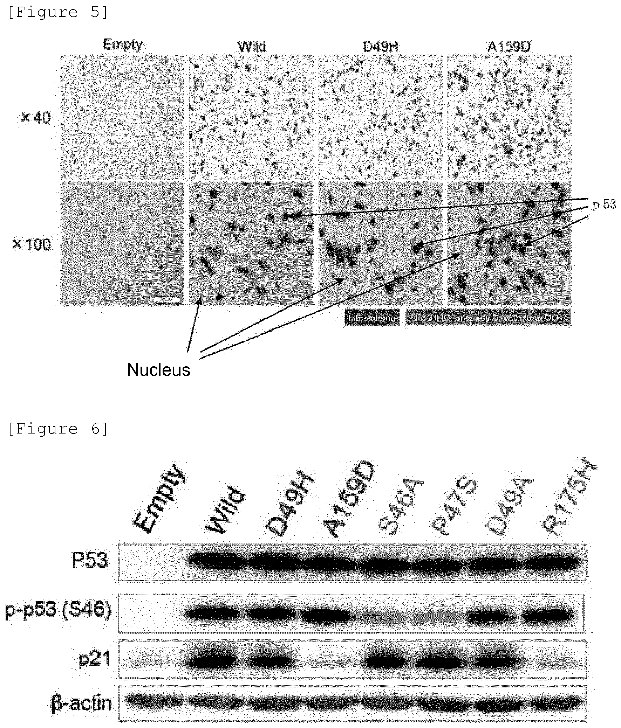

To each well of the chamber slide where transfection was thus performed, 500 μL of 4% paraformaldehyde solution was dispensed, and the chamber slide was left standing at room temperature for 30 minutes to fix the cells. After removal of the paraformaldehyde solution, the chamber slide was washed three times with PBS. Then, 500 μL of 0.1% Triton-X solution was dispensed to each well, and the chamber slide was left standing at room temperature for 5 minutes to permeabilize the cells. The chamber slide was washed three times with PBS. Then, 5% horse serum solution was dispensed to each well, and the chamber slide was left standing at room temperature for 5 minutes for blocking. An anti-p53 monoclonal antibody (Clone DO-7, manufactured by Agilent Technologies, Inc.) was diluted 100-fold with Antibody Diluent (manufactured by Agilent Technologies, Inc.) solution to provide a primary antibody solution. 200 μL of the primary antibody solution was dispensed to each well. Then, the chamber slide was left standing at room temperature for 12 hours or longer for antigen-antibody reaction. The chamber slide was washed three times with PBS. Then, a HRP (horseradish peroxidase)-labeled secondary antibody Envision+ System-HRP Labelled Polymer Anti-mouse (manufactured by Agilent Technologies, Inc.) solution was mounted onto the slide and reacted at room temperature for 1 hour. Then, the slide was washed three times with PBS and then dipped in a mixed solution of hydrogen peroxide water, DAB (diamino benzidine) and a substrate buffer solution (manufactured by Agilent Technologies, Inc.), and coloring reaction was performed for 4 to 5 minutes. After washing with PBS, nuclear staining was performed with hematoxylin (HE) for 2 to 3 minutes. The slide was washed with running water for 1 minute. The results are shown in Figure 5.

(Results)

As is evident from Figure 5, as a result of immunohistochemically staining each cell described above, the p53 protein was stained brown. A part stained blue with HE is the nucleus. Not only in the cells caused to express wild type p53 but in the cells caused to express A159D mutated p53 and D49H mutated p53, strong staining of p53 was observed in the cytoplasm as well as, particularly, in the nucleus. From this result, it was confirmed that nuclear translocation occurred in D49H mutated p53 and A159 variant p53 at the same level as in wild type p53. Thus, the decreased transcriptional activity confirmed in D49H mutated p53 and A159 mutated p53 was considered not to be ascribable to difference in the frequency of nuclear translocation but to be probably based on the functional alteration of the p53 protein caused by each mutation.

[Example 4]

[Immunoblotting of each mutated p53]

In order to evaluate influence on the phosphorylation of p53 or the expression of a gene downstream of p53, immunoblotting was performed on each mutated p53.

A cell suspension of $4 \times 10^5$ Saos-2 cells suspended in 2 mL of RPMI1640 culture medium containing 10% (v/v) heat inactivated fetal bovine serum was dispensed to each well of a 6-well plate (manufactured by Corning Inc.) and cultured for 24 hours. To 2500 ng of each DNA solution of each mutated p53 expression plasmid (D49H, A159D, S46A, P47S, D49A and R175H), a wild type p53 expression plasmid, or pReceiver-M02CT plasmid for a negative control, 5 $\mu$L of P3000 solution (manufactured by Thermo Fisher Scientific, Inc.) was added, and Opti-MEM culture medium (manufactured by Thermo Fisher Scientific, Inc.) was added to adjust the total volume to 125 $\mu$L. Then, the mixture was left standing at room temperature for 30 minutes. For each plasmid solution thus prepared, 7.5 $\mu$L of Lipofectamine 3000 solution (manufactured by Thermo Fisher Scientific, Inc.) was mixed with 117.5 $\mu$L of Opti-MEM culture medium to provide a Lipofectamine mixed solution, which was then added to each plasmid solution described above and gradually mixed therewith. Then, the mixture was left standing at room temperature for 10 minutes to prepare a transfection solution. Transfection treatment was performed by adding dropwise this whole amount, 250 $\mu$L, of each transfection solution to 1 well of the Saos-2 cells cultured on 6-well plate from the day therebefore. Then, the cells were cultured for 24 hours. Culture medium replacement was performed for the removal of the transfection solution. Then, the cells were further cultured for 24 hours. After removal of the culture medium from each well, each well was washed with 1 mL of ice cold PBS. Then, 500 $\mu$L of ice cold PBS was added again to each well. The cells were detached using Cell Lifter (manufactured by Corning Inc.), followed by centrifugation. To the obtained cell pellets, 56 $\mu$L of Lysis buffer mix (solution of M-PER mammalian protein extraction reagent supplemented with a 1/50 amount of EDTA-free Halt protease inhibitor cocktail and Halt phosphatase inhibitor cocktail (manufactured by Thermo Fisher Scientific, Inc.) was added, and the cells were left standing on ice for 45 minutes and thereby lysed to prepare a protein solution. The amount of the protein was measured using BCA protein assay reagent (manufactured by Thermo Fisher Scientific, Inc.). Lysis buffer mix was added thereto to prepare a 0.5 $\mu$g/$\mu$L protein solution. 20 $\mu$L (10 $\mu$g) of the protein solution was separated by 10% sodium dodecyl sulfate polyacrylamide gel electrophoresis (SDS-PAGE) and then transferred to a nitrocellulose sheet (manufactured by Bio-Rad Laboratories, Inc.). Subsequently, the nitrocellulose sheet was blocked by dipping in 5% skimmed milk or 5% bovine serum albumin solution prepared using Tris-buffered saline-Tween 20 (TBST, manufactured by Cell Signaling Technology Japan K.K.) at room temperature for 1 hour, and then dipped in a primary antibody solution diluted with 5% skimmed milk or 5% bovine serum albumin solution at 4°C for 12 hours or longer for antigen-antibody reaction. The nitrocellulose sheet was washed three times with TBST at ordinary temperature and then dipped in a HRP (horseradish peroxidase)-labeled secondary antibody (manufactured by Promega Corp.) solution diluted with 5% skimmed milk solution at room temperature for 1 hour. After washing five times with TBST at ordinary temperature, the signal of the antigen-antibody reaction was converted to luminescence using SuperSignal WestPico Chemiluminescent Substrate (manufactured by Thermo Fisher Scientific, Inc.) and detected using ImageQuant LAS4000 system (manufactured by GE Healthcare Japan Corp.). The results are shown in Figure 6.

(Primary antibody)

The protein expression levels of p53, p53 phosphorylated at a serine residue at position 46, and p21 were detected using primary antibodies given below. β-actin was used as a positive control.

Antibody against p53: p53 (7F5) Rabbit mAb (2527, Cell Signaling Technology Japan K.K.)
Antibody against p53 phosphorylated at a serine residue at position 46: Phospho-p53 (Ser46) Antibody (2521, Cell Signaling Technology Japan K.K.)
Antibody against p21: p21 Waf1/Cip1 (12D1) Rabbit mAb (2947, Cell Signaling Technology Japan K.K.)
Antibody against β-actin: β-Actin Antibody (C4) (sc-47778, manufactured by Santa Cruz Biotechnology, Inc.)

(Results)

As for the expression level of each p53 protein, as is evident from Figure 6, the expression levels of wild type p53 and each mutated p53 were at almost the same level.

The phosphorylation of the serine residue at position 46 has been reported as a posttranslational modification important for the induction of apoptosis by p53. Influence on the phosphorylation of the serine residue at position 46 was studied because this residue was located near D49H mutation. The expression level of the p53 protein phosphorylated at a serine residue at position 46 was at almost the same level for D49H mutated p53, A159D mutated p53, D49A mutated p53, and R175H mutated p53 as in the expression level for wild type p53. On the other hand, the expression level was decreased in S46A mutated p53 having a substitution of the serine residue at position 46 by alanine, and P47S mutated p53 having a substitution of a proline residue at position 47 by serine. From this result, it was confirmed that mutations at codons 46 and 47 are mutations that influence the phosphorylation of the serine residue at position 46, as previously reported. On the other hand, it was revealed that D49H mutation and A159D mutation are not mutations that influence the phosphorylation of the serine residue at position 46.

p21 is a major protein that is placed under the control of p53 such that the expression thereof is increased in association with the activation of p53. This protein works to arrest the cell cycle. Accordingly, each mutation was evaluated for the influence thereof on the expression of p21. As is evident from Figure 6, the expression level of the p21 protein was at almost the same level for D49H mutated p53, S46A mutated p53, P47S mutated p53, and D49A mutated p53 as in the expression level for wild type p53. The expression of the p21 protein was rarely observed in A159D mutated p53. This result is consistent with R175H mutated p53 having a loss-of-function mutation of p53 which is found with a high frequency in a plurality of cancer types. This result indicates that A159D mutation and R175H mutation influence the control of the downstream cell cycle of p53.

[Example 5]

[Cell growth test]

It has been reported that when a p53 null cell line (NCI-H1299 cells) is transfected with wild type p53 using a plasmid having neomycin resistance gene and treated with an antibiotic G418 (neomycin derivative) serving as a selection marker, the induction of apoptosis by p53 occurs, in spite of the presence of the neomycin resistance gene. A method which involves transfecting cells with a plasmid (having neomycin resistance gene) expressing an intended mutation, and then culturing the cells with an antibiotic G418 serving as a selection marker for 2 to 3 weeks, followed by evaluation on the basis of the number of colonies formed by the culture has been reported as a method for evaluating the influence of a gene mutation on cell growth. On the precondition of these findings, the following cell growth test was conducted.

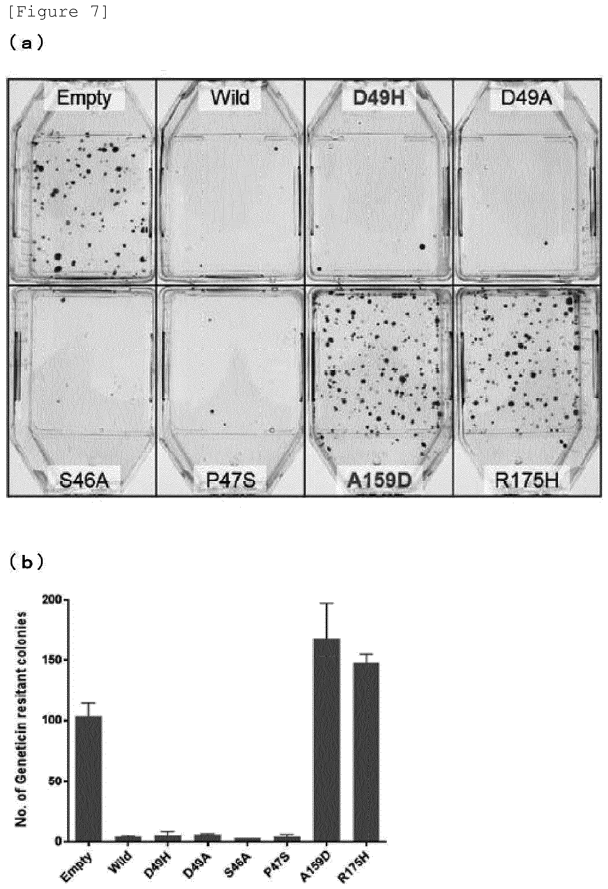

The plasmid for gene expression used in this experiment also has neomycin resistance gene as a selection marker. A cell suspension of $4 \times 10^5$ Saos-2 cells suspended in 2 mL of RPMI1640 culture medium containing 10% (v/v) heat inactivated fetal bovine serum was dispensed to each well of a 6-well plate (manufactured by Corning Inc.) and cultured for 24 hours. To 2500 ng of each DNA solution of each mutated p53 gene expression plasmid (D49H, A159D, S46A, P47S, D49A and R175H), a wild type p53 gene expression plasmid, or pReceiver-M02CT plasmid for a negative control, 5 µL of P3000 solution (manufactured by Thermo Fisher Scientific, Inc.) was added, and Opti-MEM culture medium (manufactured by Thermo Fisher Scientific, Inc.) was added to adjust the total volume to 125 µL. Then, the mixture was left standing at room temperature for 30 minutes. For each plasmid solution thus prepared, 7.5 µL of Lipofectamine 3000 solution (manufactured by Thermo Fisher Scientific, Inc.) was mixed with 117.5 µL of Opti-MEM culture medium to provide a Lipofectamine mixed solution, which was then added to each plasmid solution described above and gradually mixed therewith. Then, the mixture was left standing at room temperature for 10 minutes to prepare a transfection solution. Transfection treatment was performed by adding dropwise this whole amount, 250 µL, of each transfection solution to 1 well of the Saos-2 cells cultured on 6-well plate from the day therebefore. Then, the cells were cultured for 48 hours and then detached by the addition of Trypsin-EDTA solution (manufactured by Thermo Fisher Scientific, Inc.), and the number of cells was counted. The cell concentration was adjusted to $3.6 \times 10^4$ cells/mL. 1 mL thereof was placed in a 25-cm$^2$ flask (manufactured by Corning Inc.), to which 4 mL of RPMI1640 culture medium containing 10% (v/v) heat inactivated fetal bovine serum and 2 mg of geneticin (G418, manufactured by Thermo Fisher Scientific, Inc.) was then dispensed. This adjusted the final concentration of G418 to 0.4 mg/mL. The cells were cultured for 19 days therefrom, then washed with PBS, and fixed and stained with Crystal Violet solution, and the number of colonies was counted. The results are shown in Figures 7(a) and 7(b).

(Results)

As is evident from Figures 7(a) and 7(b), few colonies were formed for each mutated p53 having a mutation on TAD2 of p53, i.e., D49H mutated p53, D49A mutated p53, S46A mutated p53, and P47S mutated p53, as with wild type p53. On the other hand, a large number of colonies were confirmed for A159 mutated p53 and R175H mutated p53, and these numbers of colonies were confirmed to be larger than that of the Empty line.

(Discussion)

Because of a small number of colonies formed for each mutated p53, i.e., D49H mutated p53, D49A mutated p53, S46A mutated p53, and P47S mutated p53, these mutations were considered to maintain apoptosis induction-associated functions at the same level as in wild type p53. On the other hand, A159 mutated p53 and R175H mutated p53 which exhibited a large number of colonies formed were considered to lose these functions and cause no apoptosis. From the results described above, a large number of colonies were confirmed for A159D mutated p53, as with R175H mutated p53 also having a mutation on DBD. Therefore, A159D mutated p53 and R175H mutated p53 had remarkably low ability to induce apoptosis, suggesting that these mutations are likely to influence the appearance of cancer and the extension of cancer.

(Conclusion)

As described above, cells harboring A159D mutated p53 exhibited the complete loss of transcriptional activity and the promotion of cell growth. These results were consistent with the phenotype of the loss-of-function mutation of R175H of p53 which is found with a high frequency in a plurality of cancer types. The loss of p53 functions by A159D mutation confirmed in this study suggests that this mutation is a probable mutation that is one of major causes of Li-Fraumeni syndrome. On the other hand, approximately 40% significant decrease in transcriptional activity was found in D49H mutation compared with wild type p53, though marked influence on phenotype as seen in A159D mutated p53 was not observed in the D49H mutation.

[Example 6]

[Discussion on binding affinity of D49H mutated p53 and wild type p53]

Examples of the conformations of two complexes previously reported can include the following 6 types.

(1) Complex analyzed by X-ray

1) a complex with Replication Protein A (RPA)

(2) Complex analyzed by NMR

1) a complex with Tfb1 (transcription factor b1)
2) a complex with transcription factor for polymerase II (TFIIH), a general transcription factor
3) a complex with CREB-binding protein (CBP), a factor called coactivator which regulates transcription
4) a complex with p300, a factor called coactivator which regulates transcription
5) a complex with high mobility group box 1 (HMGB1), a non-histone nucleoprotein

Change in Gibbs free energy, ΔG, which is a thermodynamic potential representing the amount of work that must be done for decomposing a bound complex into component parts isolated at a sufficient distance where an extra amount of work necessary for separation is negligible, can generally be represented by the following equation 1:

[Equation 1]

$$\Delta G = G_{(complex)} - (G_{(receptor)} + G_{(ligand)}) \cdots \text{(Equation 1)}$$

Since equilibrium constant K representing binding affinity can be linked to thermodynamic quantities such as ΔG, ΔH, and ΔS by equation 2 given below, ΔG of the complexes mentioned above was estimated using results of molecular dynamic simulation to evaluate the binding affinity of the complexes. High-performance molecular dynamic simulation was performed with Linux OS machine with an installed graphic card manufactured by NVIDIA Corp. ΔG was estimated by calculating ΔH in the range of 480 to 510 nanoseconds considered to reach thermal equilibrium, and TΔS at 25°C. The binding affinity of each complex was evaluated.

[Equation 2]

$$\Delta G = -RT \ln K = \Delta H - T\Delta S \cdots \text{(Equation 2)}$$

(wherein

G:    Gibbs energy (kcal/mol)
K:    binding constant
R:    gas constant (kcal/K/mol)
T:    absolute temperature (K)
H:    enthalpy (kcal/mol)
S:    entropy (kcal/K/mol)

The evaluation of the binding affinity ($\Delta G$) of complexes with NMR as an initial structure is shown in Table 3 below.

[Table 3]

| ligand | p53 | $\Delta H$ | $T\Delta S^*$ | $\Delta G$ |
|---|---|---|---|---|
| CBP | WT | -137.4 | -78.2 | -59.2 |
| | D49H | -132.1 | -79.7 | -52.4 |
| Tfb1/p62 | WT | -41.1 | -33.3 | -7.8 |
| | D49H | -37.1 | -34.1 | -4.0 |
| HMGB1 | WT | -67.0 | -49.5 | -17.5 |
| | D49H | -80.6 | -72.2 | -8.4 |
| *25°C | | | | |

(Results)

As is evident from the Table 3, p53 (D49H)-CBP had weaker binding than that of p53 (wild type (WT))-CBP in molecular dynamic simulation and *in silico* binding affinity evaluation. It was suggested that the involvement of p53 is partly caused by the weakened binding of CBP by the introduction of D49H mutation in p53.

**Industrial Applicability**

The present invention is very useful in the medical field.

**Claims**

1.    A method for determining the presence or absence of a risk of developing a familial tumor which is caused by a germ cell mutation in p53, comprising detecting the presence or absence of a mutation to substitute alanine with aspartic acid at codon 159 of human p53 and/or a mutation to substitute aspartic acid with histidine at codon 49 of human p53 in a biological sample collected from a test subject.

2.    The method according to claim 1, wherein the mutation to substitute alanine with aspartic acid is a mutation to substitute a nucleotide sequence "GCC" encoding alanine with a nucleotide sequence "GAC" encoding aspartic acid.

3.    The method according to claim 1, wherein the mutation to substitute aspartic acid with histidine is a mutation to substitute a nucleotide sequence "GAT" encoding aspartic acid with a nucleotide sequence "CAT" encoding histidine.

4.    The method according to claim 1 or 2, wherein the mutation to substitute alanine with aspartic acid is a germ cell mutation.

5.    The method according to claim 1 or 3, wherein the mutation to substitute aspartic acid with histidine is a germ cell mutation.

6.    The method according to any one of claims 1 to 5, wherein the presence or absence of the mutation is detected using a next generation DNA sequencer.

7.    The method according to any one of claims 1 to 5, wherein the presence or absence of the mutation is detected using a Sanger method.

**Patentansprüche**

1. Verfahren zur Bestimmung des Vorliegens oder Fehlens eines Risikos der Entwicklung eines familiären Tumors, der durch eine Keimzellmutation in p53 verursacht wird, umfassend Nachweisen des Vorliegens bzw. Fehlens einer Mutation zur Substitution von Alanin durch Asparaginsäure an Codon 159 von menschlichem p53 und/oder einer Mutation zur Substitution von Asparaginsäure durch Histidin an Codon 49 von menschlichem p53 in einer einem Testindividuum entnommenen biologischen Probe.

2. Verfahren nach Anspruch 1, wobei es sich bei der Mutation zur Substitution von Alanin durch Asparaginsäure um eine Mutation zur Substitution einer Nukleotidsequenz "GCC", die Alanin codiert, durch eine Nukleotidsequenz "GAC", die Asparaginsäure codiert, handelt.

3. Verfahren nach Anspruch 1, wobei es sich bei der Mutation zur Substitution von Asparaginsäure durch Histidin um eine Mutation zur Substitution einer Nukleotidsequenz "GAT", die Asparaginsäure codiert, durch eine Nukleotidsequenz "CAT", die Histidin codiert, handelt.

4. Verfahren nach Anspruch 1 oder 2, wobei es sich bei der Mutation zur Substitution von Alanin durch Asparaginsäure um eine Keimzellmutation handelt.

5. Verfahren nach Anspruch 1 oder 3, wobei es sich bei der Mutation zur Substitution von Asparaginsäure durch Histidin um eine Keimzellmutation handelt.

6. Verfahren nach einem der Ansprüche 1 bis 5, wobei das Vorliegen oder Fehlen der Mutation unter Verwendung eines Next-Generation-DNA-Sequenzierautomaten nachgewiesen wird.

7. Verfahren nach einem der Ansprüche 1 bis 5, wobei das Vorliegen oder Fehlen der Mutation unter Verwendung eines Sanger-Verfahrens nachgewiesen wird.


**Revendications**

1. Procédé pour la détermination de la présence ou de l'absence d'un risque de développer une tumeur familiale qui est causée par une mutation de cellules germinales dans p53, comprenant la détection de la présence ou de l'absence d'une mutation pour remplacer une alanine par un acide aspartique au niveau du codon 159 de p53 humaine et/ou une mutation pour remplacer un acide aspartique par une histidine au niveau du codon 49 de p53 humaine dans un échantillon biologique prélevé sur un sujet de test.

2. Procédé selon la revendication 1, la mutation pour remplacer une alanine par un acide aspartique étant une mutation pour remplacer une séquence nucléotidique « GCC » codant pour une alanine par une séquence nucléotidique « GAC » codant pour un acide aspartique.

3. Procédé selon la revendication 1, la mutation pour remplacer un acide aspartique par une histidine étant une mutation pour remplacer une séquence nucléotidique « GAT » codant pour un acide aspartique par une séquence nucléotidique « CAT » codant pour une histidine.

4. Procédé selon la revendication 1 ou 2, la mutation pour remplacer une alanine par un acide aspartique étant une mutation de cellules germinales.

5. Procédé selon la revendication 1 ou 3, la mutation pour remplacer un acide aspartique par une histidine étant une mutation de cellules germinales.

6. Procédé selon l'une quelconque des revendications 1 à 5, la présence ou l'absence de la mutation étant détectée en utilisant un séquenceur d'ADN de prochaine génération.

7. Procédé selon l'une quelconque des revendications 1 à 5, la présence ou l'absence de la mutation étant détectée en utilisant un procédé Sanger.

[Figure 1]

## WES: IGV

Case 1

Case 2

Case 3

Blood

Tumor

Case 4

Case 5

Case 6

Blood

Tumor

[Figure 2]

## Sanger sequencing

Case 1

Case 2

Case 3

Blood

Tumor

Case 4

Case 5

Case 6

Blood

Tumor

[Figure 3]

[Figure 4]

[Figure 5]

[Figure 6]

[Figure 7]

（a）

（b）

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2003265187 A

**Non-patent literature cited in the description**

- *Oncogene,* 2004, vol. 23, 9025-9033
- **HARRIS T.D. et al.** *Science,* 2008, vol. 320, 106-109
- **KENT WJ ; SUGNET CW ; FUREY TS ; ROSKIN KM ; PRINGLE TH ; ZAHLER AM ; HAUSSLER D.** The human genome browser at UCSC. *Genome Res,* 2002, vol. 12, 996-1006, https://genome.ucsc.edu
- **SHERRY ST ; WARD MH ; KHOLODOV M ; BAKER J ; PHAN L ; SMIGIELSKI EM ; SIROTKIN K.** db-SNP: the NCBI database of genetic variation. *Nucleic Acids Res,* 2001, vol. 29, 308-311, http://www.ncbi.nlm.nih.gov/SNP
- **FORBES SA ; BEARE D ; GUNASEKARAN P ; LEUNG K ; BINDAL N ; BOUTSELAKIS H ; DING M ; BAMFORD S ; COLE C ; WARD S.** COSMIC: exploring the world's knowledge of somatic mutations in human cancer. *Nucleic Acids Res,* 2015, vol. 43, D805-811, http://cancer.sanger.ac.uk/cosmic
- **LANDRUM MJ ; LEE JM ; BENSON M ; BROWN G ; CHAO C ; CHITIPIRALLA S ; GU B ; HART J ; HOFFMAN D ; HOOVER J.** ClinVar: public archive of interpretations of clinically relevant variants. *Nucleic Acids Res,* 2016, vol. 44, D862-868, http://www.ncbi.nlm.nih.gov/clinvar
- **PETITJEAN A ; MATHE E ; KATO S ; ISHIOKA C ; TAVTIGIAN SV ; HAINAUT P ; OLIVIER M.** Impact of mutant p53 functional properties on TP53 mutation patterns and tumor phenotype: lessons from recent developments in the IARC TP53 database. *Hum Mutat,* 2007, vol. 28, 622-629, http://p53.iarc.fr
- **NAGASAKI M ; YASUDA J ; KATSUOKA F ; NARIAI N ; KOJIMA K ; KAWAI Y ; YAMAGUCHI-KABATA Y ; YOKOZAWA J ; DANJOH I ; SAITO S.** ToMMo Japanese Reference Panel Project and Yamamoto M (2015) Rare variant discovery by deep whole-genome sequencing of 1,070 Japanese individuals. *Nat Commun,* vol. 6, 8018, http://ijgvd.megabank.tohoku.ac.jp
- **HIGASA K ; MIYAKE N ; YOSHIMURA J ; OKAMURA K ; NIIHORI T ; SAITSU H ; DOI K ; SHIMIZU M ; NAKABAYASHI K ; AOKI Y.** Human genetic variation database, a reference database of genetic variations in the Japanese population. *J Hum Genet,* 2016, http://www.genome.med.kyoto-u.ac.jp/SnpDB
- **J.T. ROBINSON ; H. THORVALDSDOTTIR ; W. WINCKLER et al.** Integrative genomics viewer. *Nat. Biotechnol.,* 2011, vol. 29, 24-26
- *Oncogene,* 2007, vol. 26, 2226-2242